# EUROPEAN PATENT SPECIFICATION

(11) **EP 0 657 184 B1**
(45) Date of publication and mention of the grant of the patent: **07.11.2001**
(21) Application number: 94309057.1
(22) Date of filing: 06.12.1994
(51) Int. Cl.: A61M 25/06, A61M 37/00, A61M 5/50

(54) **Medical device**
Medizinische Vorrichtung
Dispositif médical

(30) Priority: 10.12.1993 GB 9325350; 16.03.1994 GB 9405084
(43) Date of publication of application: 14.06.1995
(60) Divisional application: 98119258.6
(73) Proprietor: The BOC Group plc, Windlesham Surrey GU20 6HJ (GB)
(72) Inventor: Nicholson, Peter James, Cricklade, Wiltshire, SN6 6JQ (GB)
(74) Representative: Ruffles, Graham Keith

(56) References cited:
- EP-A- 0 546 769
- WO-A-89/02757
- ES-A- 2 023 575
- NL-A- 8 901 124
- US-A- 4 820 267
- US-A- 5 312 345

## Description

The present invention relates to medical devices and in particular to syringes which include a hollow needle having a sharp distal end for piercing the skin of a patient.

The existence of infectious diseases such as AIDS and hepatitis has highlighted the danger to which medical personnel may be exposed when treating patients by means of catheter devices and syringes where a sharp needle point is used to pierce the skin of the patient. Medical personnel have been infected by physical contact with or an accidental prick by an infected needle (needle stick).

In order to protect medical personnel against inadvertent needle stick a number of solutions have been developed whereby protective means incorporated within a catheter or syringe prevents physical contact with the needle after use and hence against inadvertent needle stick,

Many of the developments are complicated and involve the retraction of the needle within a housing once the needle has been used. Those complications add to the expense as well as the manufacturing difficulties involved in providing adequate anti-needle stick protection.

USA patent number 4,820,267 describes a cartridge injector or pellet medicaments. The device includes a cannula supported at a proximal end thereof by a hub which slides within a tubular barrel, the barrel supporting an obturator which selectively penetrates the cannula to maintain an implanted pellet in position as the cannula is withdrawn. For single pellet dosages, the pellet is carried in the fore part of the cannula, while the case of multiple pellet dosages, the additional pellets, prior to loading, are carried in open-ended cylindrical tubes engageable with a proximal end of the hub whereby the obturator may be employed to transfer the pellet to the cannula from the sleeve which is discarded. Repositioning of the hub within the sleeve is then accomplished without disengagement of the distal end of the cannula from the tissues of the patient and additional implantations may then be performed.

EP-A 546,769 relates to a pneumoneedle through which the abdomen can be inflated with fluid such as insufflating gas. The pneumoneedle comprises a housing having inner surfaces defining an elongate cavity having a fluid passageway portion. The pneumoneedle includes a cannula having a sharpened distal end for piercing tissue. A protector is present which has a closed blunt distal end. The protector is movable relative to the cannula between a protecting position with the blunt distal end of the protector extending beyond the sharp distal end of the cannula to restrict engagement between tissue and the sharp distal end of the cannula, and retracted position with portions of the sharp distal end of the cannula extending beyond the blunt distal end of the protector to afford puncture of tissue. The pneumoneedle also includes a latching mechanism for positively locking the protector and cannula in the protecting position after the cannula has pierced tissue. Additionally, the pneumoneedle may include a release mechanism for releasing the latching mechanism when the cannula and protector are locked in the protecting position.

PCT/WO 89/02757 describes a self-blunting needle assembly comprising a hollow needle, which may be of conventional construction having a needle mouth and a needle shank terminating in a puncture tip, and has mounted within it a blunting member including an elongate probe which slidably fits within the bore of the needle shank. The probe terminates in a distal tip which is initially positioned short of the puncture tip of the needle so as not to interfere with injection of the needle either into a patient or into a connecting device. After injection, the blunting member is advanced to an extended position in which its distal tip protrudes beyond the puncture tip, thereby effectively blunting the needle. The protruding needle disables the needle from a re-use and blunts it sufficiently to prevent or greatly reduce accidental needlestick wounds. A mechanical extension member, such as a pin or fluid flowing through the needle may be used to advance the blunting member to its extended position.

It is an aim of the present invention to provide a simple but effective means for protecting the point of a needle forming part of a syringe in order to minimise the possibility of inadvertent needle stick.

According to the present invention, a syringe comprises a housing with a hollow needle extending from the distal end of the housing having a sharpened distal end for piercing the skin of a patient, the syringe including means for protecting said sharpened distal end after use to minimise the possibility of inadvertent needle stick, said means comprising a rod mounted for reciprocal movement through the hollow needle between a first needle end protection position at which an end of the rod is level with or extends beyond the sharpened distal end of the needle and a second retracted position in which said end is positioned within the hollow needle and spaced from said sharpened distal end and in which second retracted position said rod extends outwardly from the proximal end of said housing, and means effective when the rod is in the first position for maintaining the rod towards the first needle end protection position thereby preventing movement of the rod from the first needle end protection position towards the second retracted position.

Preferably, the rod is solid although it could be hollow with a blunt distal end.

In one embodiment the maintaining means includes a shoulder formed in the rod which in the second position of the rod engages behind a resilient collar forming part of the housing thereby preventing movement of the rod from the first towards the second position.

Preferably the rod is undercut to form the shoulder which engages behind the resilient collar located at the proximal end of the housing.

The maintaining means may include a resilient shoulder formed on an interior surface of the housing.

Embodiments of the invention will now be described, by way of example, reference being made to the figures of the accompanying diagrammatic drawings in which:-
Figure 1 is a side view of a medical syringe for piercing the skin of a patient to deliver a drug;
Figure 2 is a view of the syringe similar to figure 1 but showing the syringe piercing the skin of the patient;
Figure 3 is a view similar to the views of Figures 1 and 2 but showing the drug now delivered subcutaneously;
Figure 4 is a view similar to Figure 3 with the needle tip of the syringe protected and with a part sectioned to illustrate a locking feature; and
Figure 5 is an enlarged detail of the sectioned part of Figure 4.

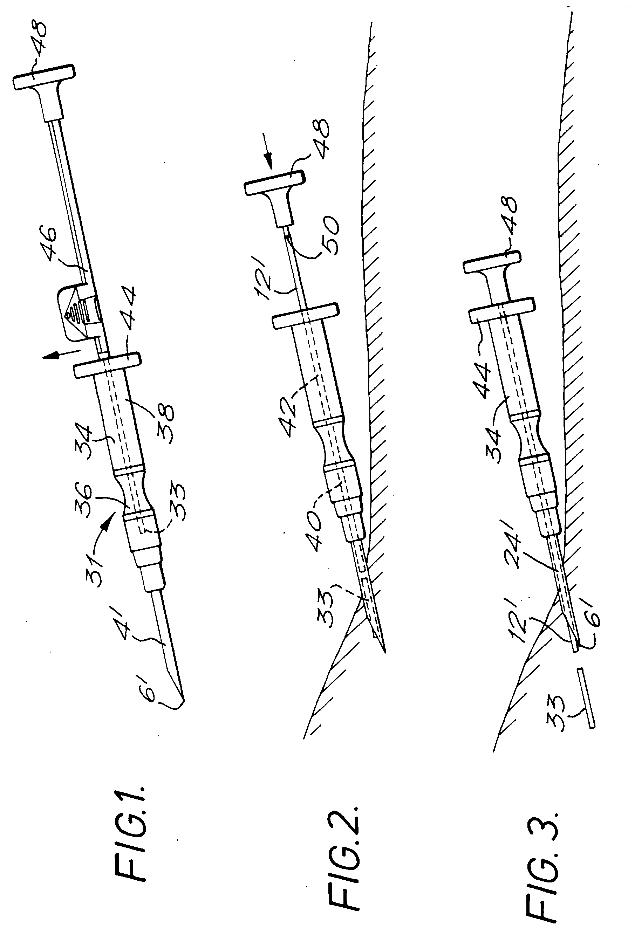

As illustrated in Figure 1 to 5, as shown, a syringe 31 for implanting a drug beneath the skin of a patient comprises a hollow needle 4' having a sharp, distal end 6' extending from the distal end of a housing 34. The housing 34 comprises two parts 36, 38 integrally joined together.
As shown parts 36, 38 have aligned passages 40, 42. A drug 33 or other substance to be administered to a patient is initially located in the passage 40 of part 36.

The part 38 includes a collar 44 at its proximal end made of resilient material.

In the ready-to-use condition a rod 12' extends outwardly from the proximal end of the part 38. The rod 12' at its proximal end is provided with a button 48. Adjacent the button 48 the surface of the rod is undercut to form a rearwardly facing annular shoulder 50.

Prior to its use, the syringe 31 is provided with a detachable spacer 46 which engages around the rod 12' between the collar 44 and the button 48 to prevent accidental movement of the rod inwardly through the housing 34. In this position the distal end of the rod 12' is located in the passage 42 of the part 38 and as previously explained the drug 33 is located in passage 40 of the part 36.

In use, when it is desired to place the drug 33 beneath the skin of a patient, the spacer 46 is removed and the sharp end 6' of the needle 4' is caused to pierce the skin of the patient as shown in Figure 2.

Next, pressure is applied to the button 48 causing the rod 12' to pass through the passages 40, 42 and engage the drug 33.

Further movement of the rod 12' through the passage 40 and the hollow needle 4' causes the drug 33 to pass along and out from the hollow needle 4 (Figure 3).

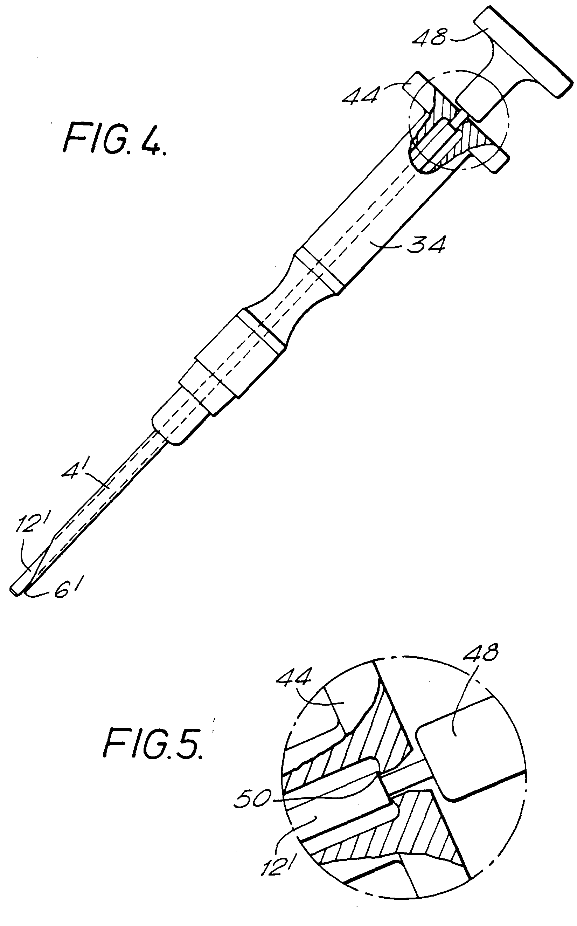

In its final position the distal end of the rod 12' extends beyond the sharp end 6' of the needle 4' and the shoulder 50 engages against the inner surface of the resilient collar 44 (Figures 4 and 5).

Thus, when the needle 4' is withdrawn from the skin of the patient its sharp end 6' is protected by the rod 12' thereby preventing accidental needle stick and enabling safe disposal of the syringe. Further, by virtue of the shoulder 50 engaging the inside surface of the resilient collar 44, the rod 12' is located in its needle end protection position and cannot be withdrawn from the needle to expose the sharp end 6'.

## Claims

1. A syringe (31) comprising a housing (34) with a hollow needle (4') extending from the distal end of the housing (34) having a sharpened distal end (6') for piercing the skin of a patient, the syringe (31) including means for protecting said sharpened distal end (6') after use to minimise the possibility of inadvertent needle stick, said means comprising a rod (12') mounted for reciprocal movement through the hollow needle (4') between a first needle end protection position at which an end (11) of the rod (12') is level with or extends beyond the sharpened distal end (6') of the needle (4') and a second retracted position in which said end (11) is positioned within the hollow needle (4') and spaced from said sharpened distal end (6') and in which second retracted position said rod (12') extends outwardly from the proximal end of said housing (34), and means effective when the rod is in the first position for maintaining the rod (12') towards the first needle end protection position thereby preventing movement A the rod (12') from the first needle end protection position towards the second retracted position.

2. A syringe (31) as claimed in claim 1, in which the rod (12') is solid.

3. A syringe (31) as claimed in claim 1 or 2, with a button (48) on the proximal end of said rod (12').

4. A syringe (31) as claimed in any preceding claim, with said rod (12') in said second retracted position.

5. A syringe (31) as claimed in claim 4, with a detachable spacer (46) engaging around the rod (12') to prevent accidental movement of the rod (12') away from said second retracted position.

6. A syringe as claimed in claim 4 or 5, wherein the housing comprises distal and proximal parts (36, 38) integrally joined with aligned passages (40, 42) for passage of the rod (12').

7. A syringe (31) as claimed in claim 6, with a substance to be administered in the passage (40) of the distal part (36).

8. A syringe (31) as claimed in claim 6 or 7, where the proximal part (38) has a collar (44) at its proximal end made of resilient material.

9. A syringe (31) as claimed in claim 8, in which the maintaining means includes a shoulder (50) formed in the rod (12') which engages behind the resilient collar (44) thereby preventing movement of the rod (12') from the second towards the first position.

10. A syringe (31) as claimed in claim 7, in which the rod (12') is undercut to form the shoulder (50) which engages behind the resilient collar (44) located at the proximal end of the housing (34).

## Patentansprüche

1. Spritze (31), die folgendes aufweist: ein Gehäuse (34) mit einer Hohlnadel (4'), die vom distalen Ende des Gehäuses (34) ausgeht und ein spitzes distales Ende (6') zum Einstechen in die Haut eines Patienten hat, wobei die Spritze (31) Mittel zum Schutz des spitzen distalen Endes (6') nach der Benutzung einschließt, um die Möglichkeit eines unbeabsichtigten Nadelstichs auf ein Minimum zu verringern, wobei die Mittel eine Stange (12') umfassen, die angebracht ist für die hin- und hergehende Bewegung durch die Hohlnadel (4') zwischen einer ersten, einer Nadelende-Schutzposition, bei der ein Ende (11) der Stange (12') bündig mit dem spitzen distalen Ende (6') der Nadel (4') ist oder über dieses hinaus vorsteht, und einer zweiten, zurückgezogenen Position, in der sich das Ende (11) innerhalb der Hohlnadel (4') befindet und mit Zwischenraum zu dem spitzen distalen Ende (6') angeordnet ist, und wobei in dieser zweiten, zurückgezogenen Position die Stange (12') vom proximalen Ende des Gehäuses (34) nach außen verläuft, und Mittel, die, wenn sich die Stange in der ersten Position befindet, wirksam sind, um die Stange (12') zu der ersten Nadelende-Schutzposition hin zu halten, um dadurch die Bewegung der Stange (12') aus der ersten, der Nadelende-Schutzposition in die zweite, zurückgezogene Position zu verhindern.

2. Spritze (31) nach Anspruch 1, bei der die Stange (12') massiv ist.

3. Spritze (31) nach Anspruch 1 oder 2 mit einem Knopf (48) am proximalen Ende der Stange (12').

4. Spritze (31) nach einem der vorhergehenden Ansprüche mit der Stange (12') in der zweiten, zurückgezogenen Position.

5. Spritze (31) nach Anspruch 4, bei dem ein abnehmbares Abstandselement (46) um die Stange (12') im Eingriff ist, um die zufällige Bewegung der Stange (12') heraus aus der zweiten, zurückgezogenen Position zu verhindern.

6. Spritze (31) nach Anspruch 4 oder 5, bei der das Gehäuse einen distalen und einen proximalen Teil (36, 38) aufweist, die integriert, mit ausgerichteten Durchgängen (40, 42) für den Durchgang der Stange (12') verbunden sind.

7. Spritze (31) nach Anspruch 6 mit einer zu verabreichenden Substanz im Durchgang (40) des distalen Teils (36).

8. Spritze (31) nach Anspruch 6 oder 7, bei welcher der proximale Teil (38) an seinem proximalen Ende einen Bund (44) hat, der aus einem elastischen Material hergestellt wird.

9. Spritze (31) nach Anspruch 8, bei der die Haltemittel einen Absatz (50) einschließen, der in der Stange (12') gebildet wird, der hinter dem elastischen Bund (44) zum Eingriff kommt, um dadurch die Bewegung der Stange (12') aus der zweiten hin zur ersten Position zu verhindern.

10. Spritze (31) nach Anspruch 7, bei der die Stange (12') unterschnitten ist, um den Absatz (50) zu bilden, der hinter dem elastischen Bund (44) zum Eingriff kommt, der sich am proximalen Ende des Gehäuses (34) befindet.

## Revendications

1. Seringue (31) comprenant un boîtier (34) avec une aiguille creuse (4') s'étendant à partir de l'extrémité distale du boîtier (34), comportant une extrémité distale tranchante (6') pour percer la peau d'un patient, la seringue (31) englobant un moyen pour protéger ladite extrémité distale tranchante (6') après l'utilisation pour réduire au minimum le risque d'une piqûre d'aiguille accidentelle, ledit moyen comprenant une tige (12') montée de sorte à effectuer un mouvement de va-et-vient à travers l'aiguille creuse (4'), entre une première position de protection de l'extrémité de l'aiguille au niveau de laquelle une extrémité (11) de la tige (12) est agencée au niveau de l'extrémité distale tranchante (6') de l'aiguille (4') ou s'étend au-delà de celle-ci, et une deuxième position rétractée dans laquelle ladite extrémité (11) est positionnée dans l'aiguille creuse (4') et espacée de ladite extrémité distale tranchante (6'), ladite tige (12') s'étendant dans ladite deuxième position rétractée vers l'extérieur de l'extrémité proximale dudit boîtier (34), et un moyen efficace pour maintenir la tige (12') vers la première position de protection de l'extrémité de l'aiguille lorsque la tige se trouve dans la première position, empêchant ainsi le déplacement de la tige (12') de la première position de protection de l'extrémité de l'aiguille vers la deuxième position rétractée.

2. Seringue (31) selon la revendication 1, dans laquelle la tige (12') est solide.

3. Seringue (31) selon les revendications 1 ou 2, comportant un bouton (48) sur l'extrémité proximale de ladite tige (12').

4. Seringue (31) selon l'une quelconque des revendications précédentes, ladite tige (12') étant agencée dans ladite deuxième position rétractée.

5. Seringue (31) selon la revendication 4, comportant un élément d'espacement amovible (46) s'engageant autour de la tige (12') pour empêcher un déplacement accidentel de la tige (12') à l'écart de ladite deuxième position rétractée.

6. Seringue selon les revendications 4 ou 5, dans laquelle le boîtier comprend des parties distale et proximale (36, 38), reliées intégralement avec des passages alignés (40, 42) pour permettre le passage de la tige (12').

7. Seringue (31) selon la revendication 6, contenant une substance devant être administrée dans le passage (40) de la partie distale (36).

8. Seringue (31) selon les revendications 6 ou 7, dans laquelle la partie proximale (38) comporte un collier (44) au niveau de son extrémité proximale, composé d'un matériau élastique.

9. Seringue (31) selon la revendication 8, dans laquelle le moyen de retenue englobe un épaulement (50) formé dans la tige (12') s'engageant derrière le collier élastique (44), empêchant ainsi le déplacement de la tige (12') de la deuxième position vers la première position.

10. Seringue (31) selon la revendication 7, dans laquelle la tige (12') est entaillée pour former un épaulement (50) s'engageant derrière le collier élastique (44) agencé au niveau de l'extrémité proximale du boîtier (34).
